# EUROPEAN PATENT APPLICATION

(11) **EP 4 230 657 A1**
(43) Date of publication of application: **23.08.2023**
(21) Application number: 21882789.7
(22) Date of filing: 18.10.2021
(51) Int. Cl.: C07K 19/00, A61K 39/215, A61K 39/39, C07K 14/11, C07K 14/165, C12N 9/24

(54) **FUSION PROTEIN AND VACCINE**

(30) Priority: 19.10.2020 JP 2020175599
(71) Applicant: The Research Foundation for Microbial Diseases of Osaka University, Suita-shi, Osaka 565-0871 (JP)
(72) Inventor: YOSHIOKA, Yasuo, Suita-shi, Osaka 565-0871 (JP)
(74) Representative: Müller-Boré & Partner Patentanwälte PartG mbB
(86) International application number: PCT/JP2021/038496
(87) International publication number: WO 2022/085648

(57) **Abstract**

The present invention provides a new component that is useful as a SARS-CoV-2 vaccine antigen that uses as a target a receptor binding domain of SARS-CoV-2. The present invention contains the fusion protein, which includes hemagglutinin and a receptor binding domain of SARS-CoV-2, and a vaccine containing the fusion protein.

## Description

### TECHNICAL FIELD

The present invention relates to a fusion protein that is useful as a vaccine antigen against an infection caused by SARS-CoV-2 and the like, and a vaccine using the fusion protein.

### BACKGROUND ART

The infection with the novel coronavirus (SARS-CoV-2) as a pathogen that occurred in Wuhan, China in 2019 (COVID-19) has caused a pandemic worldwide and is a major social problem.

Vaccine development for SARS-CoV-2 is progressing rapidly around the world. The only vaccine that has been approved as of October 2020 is Sputnik V that has been approved in Russia (Non-Patent Document 1).

### PRIOR ART DOCUMENT

### NON-PATENT DOCUMENT

Non-Patent Document 1: THE LANCET, VOLUME 396, ISSUE 10255, P887-897, SEPTEMBER 26, 2020

### SUMMARY OF THE INVENTION

### PROBLEMS TO BE SOLVED BY THE INVENTION

Sputnik V is still in the stage of conducting clinical trials, and there are raised questions about safety and efficacy. In addition, even if vaccination is started, there is still a risk that the effect is small or heavy side effects are found, and it is not clear whether the vaccination is a decisive blow to prevent the spread of the infection. Thus, additional options for vaccines against SARS-CoV-2 are desired.

In the development of a vaccine against SARS-CoV-2, it is considered effective to target a receptor-binding domain (RBD), which plays a particularly important role in infection, of a spike protein. However, since RBD alone cannot exhibit an antibody titer-inducing property, in the case of making a vaccine, there is a limitation on the formulation that requires an adjuvant.

Therefore, an object of the present invention is to provide a new component that is useful as a vaccine antigen against an infection caused by SARS-CoV-2 and the like.

### MEANS FOR SOLVING THE PROBLEM

As a result of intensive studies, the present inventor has found that a fusion protein obtained by fusing an antigen against which a human already has antibodies, specifically, hemagglutinin and/or neuraminidase, to a receptor-binding domain of SARS-CoV-2 can exhibit an antibody titer-inducing property alone. In other words, the present inventor has found that the antibody titer-inducing property can be exhibited by fusing an antigen of a virus and/or a bacterium to an antigen against which a vaccinated subject has antibodies in the body. The present invention has been completed by further conducting studies based on this finding.

In other words, the present invention provides inventions of the following embodiments.
Item 1. A fusion protein containing a receptor-binding domain of SARS-CoV-2 and hemagglutinin and/or neuraminidase.
Item 2. The fusion protein according to item 1, wherein the hemagglutinin is H1 type, H3 type, H5 type, and/or H7 type.
Item 3. The fusion protein according to item 1 or 2, which is used as a vaccine antigen against SARS-CoV-2.
Item 4. A fusion protein that is used as a vaccine antigen and containing an antigen of a virus and/or a bacterium and an antigen against which a vaccinated subject has antibodies in a body.
Item 5. A vaccine containing the fusion protein according to any one of items 1 to 4.
Item 6. The vaccine according to item 5, which does not contain an adjuvant.
Item 7. The vaccine according to item 5 or 6, which is administered to a subject having antibodies against an influenza virus.
Item 8. The vaccine according to any one of items 5 to 7, which is administered by injection.
Item 9. The vaccine according to any one of items 5 to 7, which is administered nasally.

### ADVANTAGES OF THE INVENTION

According to the present invention, there are provided a new component that is useful as a vaccine antigen against a target for SARS-CoV-2 infection prevention and/or onset prevention, and a vaccine using the same.

### BRIEF DESCRIPTION OF THE DRAWINGS

Fig. 1 shows results of induction of RBD-specific IgG in plasma by Test Example 1 (subcutaneous administration).
Fig. 2A shows results of induction of RBD-specific IgG in plasma by Test Example 2 (nasal administration).
Fig. 2B shows results of induction of RBD-specific IgA in nasal wash by Test Example 2 (nasal administration).
Fig. 3 shows results of induction of RBD-specific IgG in plasma by Test Example 3 (subcutaneous administration).
Fig. 4 shows results of induction of RBD-specific IgG in plasma by Test Example 4 (subcutaneous administration).
Fig. 5 shows results of evaluation of neutralizing activity in plasma by Test Example 5 (subcutaneous administration).
Fig. 6 shows changes in body weight of mice by Test Example 6 (subcutaneous administration).
Fig. 7 shows results of induction of RBD-specific IgG in plasma by Test Example 7 (subcutaneous administration).
Fig. 8 shows results of induction of RBD-specific IgG in plasma by Test Example 8 (subcutaneous administration).
Fig. 9 shows results of induction of RBD-specific IgG in plasma by Test Example 9 (nasal administration).
Fig. 10 shows neutralizing activity by plasma in Fig. 9.
Fig. 11 shows results of induction of RBD-specific IgA in nasal wash by Test Example 11 (nasal administration).
Fig. 12 shows SARS-CoV-2 in nasal wash by Test Example 12 (nasal administration).
Fig. 13 shows results of induction of RBD-specific IgA in nasal wash by Test Example 13 (nasal administration).

### EMBODIMENTS OF THE INVENTION

### 1. Fusion protein

A fusion protein of the present invention is characterized by containing an antigen of a virus and/or a bacterium and an antibody possessed by a human who is a vaccinated subject in the body. A particularly preferred form of the fusion protein of the present invention is characterized by containing a receptor-binding domain of SARS-CoV-2 and hemagglutinin and/or neuraminidase.

Examples of the antigen of a virus and/or a bacterium that is a target for infection prevention constituting the fusion protein of the present invention include a receptor-binding domain (RBD) protein of coronavirus; PspA derived from pneumococcus; an F protein and a G protein derived from RS virus, and an env protein derived from HIV, and preferably include a receptor-binding domain (RBD) protein of coronavirus.

Here, the coronavirus is morphologically spherical with a diameter of about 100 to 200 nm and has protrusions on the surface. The coronavirus is classified into Nidovirales, Coronavirinae, Coronaviridae. In the envelope of the lipid bilayer membrane, there is a genome of positive-stranded single-stranded RNA wound around a nucleocapsid protein (also referred to as a nucleocapsid), and a spike protein (hereinafter also referred to as "spike"), an envelope protein (hereinafter also referred to as "envelope"), and a membrane protein are arranged on the surface of the envelope. The size of the viral genome is about 30 kb, the longest among RNA viruses.

Coronaviruses are classified into groups of alpha, beta, gamma, and delta according to genetic characteristics. As coronaviruses infecting humans, 4 types of human coronaviruses 229E, OC43, NL63, and HKU-1 as causative viruses of cold, and severe acute respiratory syndrome (SARS) coronavirus that occurred in 2002 and Middle East respiratory syndrome (MERS) coronavirus that occurred in 2012, which cause serious pneumonia, are known. Human coronaviruses 229E and NL63 are classified into the alpha-coronavirus genus, and human coronaviruses OC43, HKU-1, SARS coronavirus, and MERS coronavirus are classified into the beta-coronavirus genus. In the present invention, the coronavirus is preferably coronavirus belonging to the beta-coronavirus genus, and more preferably SARS coronavirus.

SARS-CoV-2 has been isolated and identified as a causative virus of the novel coronavirus infection that occurred in Wuhan in 2019. SARS-CoV-2 has been mutated repeatedly from the early Wuhan strain, and mutant strains such as a strain detected in the United Kingdom, a strain detected in South Africa, and a strain detected in India have been found. There is also a possibility that there are a mutant strain that have not yet been detected or new mutant strains will occur in the future. Therefore, in the present invention, the SARS coronavirus is not limited to the above SARS-CoV-2 strain, and includes other SARS-CoV-2 mutant strains that will be newly detected in the future.

The receptor-binding domain (RBD) of SARS-CoV-2 is a region of a spike protein of SARS-CoV-2 that plays a particularly important role in infection.

The RBD of SARS-CoV-2 constituting a preferred fusion protein of the present invention includes the following (1-1) to (1-3):
(1-1) a polypeptide consisting of an amino acid sequence from position 319 to 541 of SEQ ID NO: 1,
(1-2) a polypeptide in which one or several amino acid residues are substituted, added, inserted, and/or deleted in an amino acid sequence from position 319 to 541 of SEQ ID NO: 1 and that forms a complex with angiotensin-converting enzyme 2 (ACE2), and
(1-3) a polypeptide that has a sequence identity of 80% or more with an amino acid sequence from position 319 to 541 of SEQ ID NO: 1 and forms a complex with angiotensin-converting enzyme 2 (ACE2).

In the polypeptide of (1-1), SEQ ID NO: 1 represents an amino acid sequence of a spike protein (YP_009724390.1) of SARS-CoV-2 (hereinafter also referred to as "specific SARS-Cov-2") listed as NC_045512 in NCBI, and an amino acid sequence from position 319 to 541 thereof corresponds to the RBD.

The polypeptides of (1-1) to (1-3) include not only polypeptides obtained by artificial substitution but also polypeptides originally having such an amino acid sequence.

The polypeptides of (1-2) and (1-3) are allowed to have a difference in amino acid sequence as compared with the polypeptide of (1-1) as long as they have the same complex-forming ability with ACE2 as the polypeptide of (1-1), and are also referred to as a different form of the polypeptide of (1-1).

The amino acid difference in the polypeptide of (1-2) may include only one kind of difference (e.g., substitution) among substitution, addition, insertion, and deletion, or may include two or more kinds of the differences (e.g., substitution and insertion). In the polypeptide of (1 -2), the number of amino acid differences may be 1 or several, and is, for example, 1 to 15, preferably 1 to 10, more preferably 1 to 8, still more preferably 1 to 7 or 1 to 6, still more preferably 1 to 5 or 1 to 4, still more preferably 1 to 3, particularly preferably 1 or 2, and most preferably 1.

The sequence identity between the amino acid sequence of the polypeptide of (1-3) and the amino acid sequence shown in (1-1) may be 80% or more, but is preferably 85% or more, more preferably 90% or more, still more preferably 95% or more, still more preferably 96% or more, still more preferably 97% or more, particularly preferably 98% or more, and most preferably 99% or more.

Here, in the polypeptide of (1-3), the "sequence identity" refers to a value of identity of an amino acid sequence obtained by bl2seq program of BLASTPACKAGE [sgi32 bit edition, Version 2.0.12; available from National Center for Biotechnology Information (NCBI)] (Tatiana A. Tatsusova, Thomas L. Madden, FEMS Microbiol. Lett., Vol. 174, p247?250, 1999). Parameters may be set to Gap insertion Cost value: 11 and Gap extension Cost value: 1.

In the polypeptides of (1-2) and (1-3), it is considered that position 487 (asparagine), position 493 (glutamine), position 498 (glutamine), and position 505 (tyrosine) in the amino acid sequence shown in SEQ ID NO: 1 contribute to interactions with main hot spot amino acid residues of an ACE2 receptor, and thus it is desirable not to introduce substitutions or deletions into these sites.

When an amino acid substitution is introduced into the polypeptides of (1-2) and (1-3), a conservative substitution is mentioned as an embodiment of the amino acid substitution. In other words, in the polypeptides of (1-2) and (1-3), examples of the amino acid substitution introduced into the amino acid sequence shown in SEQ ID NO: 1 include a substitution with another non-polar amino acid if the amino acid before substitution is a non-polar amino acid, a substitution with another non-charged amino acid if the amino acid before substitution is a non-charged amino acid, a substitution with another acidic amino acid if the amino acid before substitution is an acidic amino acid, and a substitution with another basic amino acid if the amino acid before substitution is a basic amino acid.

An antigen against which a vaccinated subject has antibodies in the body constituting the fusion protein of the present invention can be fused to the antigen of a virus and/or a bacterium that is a target for infection prevention and/or onset prevention described above, whereby the fusion protein of the present invention can be provided with or improved in a property of inducing specific antibodies against the antigen of a virus and/or a bacterium that is a target for infection prevention and/or onset prevention.

The antigen against which a vaccinated subject has antibodies in the body may be an antigen of antibodies that has been possessed by the subject in the body by past infection or vaccination. Such an antigen may be an antigen against a virus and/or a bacterium different from the virus and/or bacterium, which should prevent the infection and/or onset of the disease, and examples thereof include CRM197 (particularly, when the fusion protein of the present invention is used for a vaccine antigen of a transdermal vaccine) and IgA retained in the upper respiratory tract (particularly, when the fusion protein of the present invention is used for a vaccine antigen of a nasal vaccine). Preferred examples of such an antigen include hemagglutinin and neuraminidase.

Each of hemagglutinin and neuraminidase constituting the preferred fusion protein of the present invention can be fused to the above RBD, whereby the fusion protein of the present invention can be provided with or improved in a property of inducing specific antibodies against the RBD of SARS-CoV-2 (hereinafter also referred to as "RBD-specific antibody titer-inducing property"). The hemagglutinin and/or neuraminidase used for the fusion protein of the present invention is not particularly limited, and one or more can be selected from those known as glycoproteins present on the surface of a virus. From the viewpoint of providing the fusion protein of the present invention with a further improved RBD-specific antibody-inducing property, hemagglutinin is preferred, hemagglutinin derived from an influenza virus is more preferred, hemagglutinin (H1 type hemagglutinin to H16 type hemagglutinin) derived from an influenza A virus is still more preferred, H1 type, H3 type, H5 type, or H7 type hemagglutinin is still more preferred, and H1 type hemagglutinin is most preferred. Amino acid sequences of these hemagglutinin and neuraminidase can be obtained from the official database.

Preferred amino acid sequences of hemagglutinin used for the fusion protein of the present invention include the following (2 -1) to (2-3):
(2-1) a polypeptide consisting of an amino acid sequence from position 18 to 523 of SEQ ID NO: 2,
(2-2) a polypeptide in which one or several amino acid residues are substituted, added, inserted, and/or deleted in an amino acid sequence from position 18 to 523 of SEQ ID NO: 2 and that improves an RBD-specific antibody titer-inducing property, and
(2-3) a polypeptide that has a sequence identity of 80% or more with an amino acid sequence from position 18 to 523 of SEQ ID NO: 2 and improves an RBD-specific antibody titer-inducing property.

In the polypeptide of (2-1), SEQ ID NO: 2 represents an amino acid sequence of an H1 subunit of influenza A H1N1 type (A/California/04/2009) listed as ACV82259.1 in NCBI, and an amino acid sequence from position 18 to 523 excluding the leader sequence of the N-terminal portion and the extracellular domain of the C-terminal portion thereof is used as the amino acid sequence of the polypeptide of (2-1).

The polypeptides of (2-1) to (2-3) include not only polypeptides obtained by artificial substitutions but also polypeptides originally having such an amino acid sequence.

The polypeptides of (2-2) and (2-3) are allowed to have differences in amino acid sequence as compared with the polypeptide of (2-1) as long as they improve the RBD-specific antibody titer-inducing property, and are also referred to as a different form of the polypeptide of (2-1). The phrase "improving the RBD-specific antibody titer-inducing property" means that an RBD-specific antibody titer in the case where a fusion protein is formed with the above RBD is made higher than an RBD-specific antibody titer in the case of the RBD alone. In a preferred embodiment of "improving the RBD-specific antibody titer-inducing property", the RBD-specific antibody titer in the case where a fusion protein is formed with the above RBD is made equal to or higher than that of the polypeptide of (2-1), more preferably 80% or more, still more preferably 90% or more, still more preferably 95% or more, and still more preferably 100% or more, with an RBD-specific antibody titer induced when the polypeptide of (2-1) is fused to the RBD being 100%.

The amino acid difference in the polypeptide of (2-2) may include only one kind of difference (e.g., substitution) among substitution, addition, insertion, and deletion, or may include two or more kinds of the differences (e.g., substitution and insertion). In the polypeptide of (2-2), the number of amino acid differences may be 1 or several, and is, for example, 1 to 15, preferably 1 to 10, more preferably 1 to 8, still more preferably 1 to 7 or 1 to 6, still more preferably 1 to 5 or 1 to 4, still more preferably 1 to 3, particularly preferably 1 or 2, and most preferably 1.

The sequence identity in (2-3) is the same as the sequence identity in (1-3).

The embodiment of the amino acid substitutions when amino acid substitutions are introduced into the polypeptides of (2-2) and (2-3) is the same as the embodiment of the amino acid substitutions in the polypeptides of (1-2) and (1-3).

The fusion protein of the present invention may have other structures in addition to the antigen of a virus and/or a bacterium that is a target for infection prevention described above (preferably, the above RBD) and the antigen against which a vaccinated subject has antibodies in the body (preferably, hemagglutinin and/or neuraminidase). Examples of the other structures include a linker peptide that binds the antigen of a virus and/or a bacterium that is a target for infection prevention described above (the above RBD) and the antigen against which a vaccinated subject has antibodies in the body described above (preferably hemagglutinin or neuraminidase, particularly preferably hemagglutinin). Examples of the linker peptide include linker peptides consisting of the following amino acid sequences, preferably GS linkers such as Linker Example 1 and Linker Example 2, more preferably Linker Example 1, and still more preferably a linker in which n = 1 and m = 1 in Linker Example 1.
Linker Example 1: [(G)nS]m; n = 1 to 5, m = 1 to 5
Linker Example 2: [(G)nS]mGG; n = 1 to 5, m = 1 to 5
Linker Example 3: (G)n; n = 5 to 10
Linker Example 4: (EAAAK)n; n = 1 to 4
Linker Example 5: (PA)n; n = 2 to 34

As described above, since the fusion peptide of the present invention can be provided with or improved in a property of inducing specific antibodies against an antigen of a virus and/or a bacterium that is a target for infection prevention, the fusion peptide can be used as a vaccine antigen against the virus and/or the bacterium that is a target for infection prevention. In particular, when the fusion protein of the present invention is a fusion protein containing RBD of SARS-CoV-2 and hemagglutinin and/or neuraminidase, the fusion protein can be used as a vaccine antigen against SARS-CoV-2 since the fusion protein can be provided with or improved in the RBD-specific antibody titer-inducing property.

### 2. Method for producing fusion protein

A method for producing the fusion protein described in the above "1. Fusion protein" includes step 1 of introducing DNA encoding an antigen of a virus and/or a bacterium that is a target for infection prevention and/or onset prevention and DNA encoding an antigen against which a vaccinated subject has antibodies in the body into host cells to obtain a transformant; step 2 of culturing the transformant; and step 3 of recovering from the cultured transformant product containing the receptor-binding domain and the hemagglutinin and/or neuraminidase.

A preferred embodiment of the method for producing the fusion protein described above includes step 1 of introducing DNA encoding a receptor-binding domain of SARS-CoV-2 and DNA encoding hemagglutinin and/or neuraminidase into host cells to obtain a transformant; step 2 of culturing the transformant; and step 3 of recovering from the cultured transformant product containing the receptor-binding domain and the hemagglutinin and/or neuraminidase.

In step 1, DNA encoding an antigen of a virus and/or a bacterium that is a target for infection prevention and DNA encoding an antigen against which a vaccinated subject has antibodies in the body (preferably, DNA encoding a receptor-binding domain (RBD) of SARS-CoV-2 and DNA encoding hemagglutinin and/or neuraminidase) can be introduced by inserting them in an appropriate form into expression vectors and incorporating the obtained recombinant vectors into host cells. Preferably, recombinant vectors containing DNA encoding RBD and DNA encoding hemagglutinin and/or neuraminidase (preferably containing DNA encoding RBD, DNA encoding a linker peptide, and DNA encoding hemagglutinin and/or neuraminidase) can be produced and introduced into host cells.

In a method for designing and synthesizing the DNA described above, base sequence information of each gene of a target polypeptide, specifically, an antigen of a virus and/or a bacterium that is a target for infection prevention and an antigen against which a vaccinated subject has antibodies in the body (preferably, base sequence information of each gene encoding RBD and hemagglutinin and/or neuraminidase) is acquired from a known database (GenBank or the like), a target gene is cloned using a known method, base sequence information is acquired by performing base sequence analysis, nucleic acid is extracted from each microorganism having a target antigen of a virus and/or a bacterium that is a target for infection prevention and a target antigen against which a vaccinated subject has antibodies in the body (preferably, each microorganism having RBD and hemagglutinin and/or neuraminidase) based on the obtained base sequence information, a gene is acquired using a known means such as PCR, and a fusion gene (hybrid gene) in which the respective genes are artificially linked can be produced.

When the target polypeptide has a mutation, a mutation is introduced into a gene encoding the polypeptide. A method for introducing a specific mutation into a specific site of a base sequence is known, and for example, site-directed mutation introduction of DNA, site-directed mutagenesis, and the like (specifically, a Kunkel method, a Gapped duplex method, a megaprimer PCR method, and the like) can be used.

The above DNA is preferably one in which the codon usage frequency is optimized for a host, and more preferably DNA in which the codon usage frequency is optimized for mammalian cells. As an index representing the codon usage frequency, a total of the host optimal codon usage frequency of each codon may be adopted. The optimal codon is defined as a codon having the highest usage frequency among codons corresponding to the same amino acid.

The recombinant vector is obtained by inserting the above DNA into an appropriate expression vector by restriction enzyme cleavage, ligation, and the like. The expression vector is not particularly limited, and a vector constructed for genetic recombination from a phage, a plasmid, or a virus capable of autonomously replicating in a host is suitable.

The recombinant vector includes a control factor such as a promoter operably linked to the above DNA. The control factor typically includes a promoter, but may further include a transcription element such as an enhancer, a CCAAT box, a TATA box, or an SPI site as necessary. In addition, operably linking means that various control factors such as a promoter and an enhancer that control the above DNA are linked to the above DNA in a state in which they can operate in a host cell.

The host is not particularly limited as long as the recombinant vector is stable, can autonomously replicate, and can express a trait of an exogenous gene, but suitable examples thereof include bacteria belonging to the genus Escherichia such as Escherichia coli, the genus Bacillus such as Bacillus subtilis, the genus Pseudomonas such as Pseudomonas putida, and the like; and yeast, and the host may be an animal cell such as a 293 cell, a 293T cell, an Expi293F cell, or a CHO cell, an insect cell, a plant cell, and the like. Among them, Escherichia coli is particularly preferable.

Conditions for introducing the recombinant vector into a host cell in order to obtain a transformant may be appropriately set according to the type of the host cell, and the like. When the host cell is a bacterium, examples of the conditions include a method using a competent cell by calcium ion treatment and an electroporation method. When the host cell is yeast, examples of the conditions include an electroporation method, a spheroplast method, and a lithium acetate method. When the host cell is an animal cell, examples of the conditions include an electroporation method, a calcium phosphate method, and a lipofection method. When the host cell is an insect cell, examples of the conditions include a calcium phosphate method, a lipofection method, and an electroporation method. When the host cell is a plant cell, examples of the conditions include an electroporation method, an Agrobacterium method, a particle gun method, and a PEG method.

Whether or not the above recombinant vector is incorporated into a host cell can be confirmed by a known method such as a PCR method, a Southern hybridization method, or a Northern hybridization method.

In step 2, by culturing the transformant, a fusion protein containing an antigen of a virus and/or a bacterium that is a target for infection prevention and/or onset prevention and an antigen against which a vaccinated subject has antibodies in the body (preferably, a fusion protein containing RBD and hemagglutinin and/or neuraminidase) can be produced as product.

Conditions for culturing the transformant may be appropriately set in consideration of nutritional physiological properties of the host, and liquid culture is preferable. In the case of industrial production, aerobic stirring culture is preferable. As a nutrient source of a medium, those required for growth of the transformant can be used. A culture temperature can be appropriately set within a range in which the transformant can grow and the transformant produces a fusion protein.

In step 3, the above product is recovered. A culture may be a culture supernatant, or a cultured cell, or a cultured bacterial cell or a disrupted product thereof. When a target fusion protein (product) is produced in a host cell, the product can be extracted by disrupting the host cell. When the target fusion protein (product) is produced outside the host cell, a culture solution can be used as it is, or the host cell can be removed by centrifugation or the like. Thereafter, the product can be recovered by isolation and/or purification using a general biochemical method used for isolation and purification of proteins, for example, ammonium sulfate precipitation, gel filtration, ion exchange chromatography, affinity chromatography, or the like alone or in appropriate combination.

### 3. Vaccine

Since the fusion protein of the present invention can be provided with or improved in a property of inducing specific antibodies against an antigen of a virus and/or a bacterium that is a target for infection prevention and/or onset prevention (preferably, an RBD-specific antibody titer-inducing property of SARS-CoV-2) as described above, and is useful as a vaccine antigen against a virus and/or a bacterium that is a target for infection prevention and/or onset prevention (preferably, SARS-CoV-2), the present invention also provides a vaccine containing the above fusion protein.

The vaccine of the present invention can contain other components such as buffer, tonicity agents, soothing agents, preservatives, and antioxidants according to the purpose and use, and the like, in addition to the above fusion protein as a vaccine antigen against a virus and/or a bacterium (preferably, SARS-CoV-2) that is a target for infection prevention and/or onset prevention.

Examples of the buffer include such as phosphate, acetate, carbonate, and citrate. Examples of the tonicity agents include sodium chloride, glycerin, and D-mannitol. Examples of the soothing agents include benzyl alcohol. Examples of the preservatives include thimerosal, para-hydroxybenzoates, phenoxyethanol, chlorobutanol, benzyl alcohol, phenethyl alcohol, dehydroacetic acid, sorbic acid, antibiotics, synthetic antibacterial agents, and the like. Examples of the antioxidants include sulfite and ascorbic acid.

In addition to the above components, the vaccine of the present invention can also contain light-absorbing dye (riboflavin, adenine, adenosine, and the like), stabilizers (chelating agents, reducing agents, and the like), carbohydrate (sorbitol, lactose, mannitol, starch, sucrose, glucose, dextran, and the like), casein digest, various vitamins, flavoring agents, adjuvants, and the like.

Since the fusion protein of the present invention described above can be provided with or improved in a specific antibody titer-inducing property against a virus and/or a bacterium that is a target for infection prevention and/or onset prevention (preferably, an RBD-specific antibody titer-inducing property of SARS-CoV-2), the specific antibody titer-inducing property can be effectively induced even if no adjuvant is blended in the vaccine. Therefore, preferred embodiments of the vaccine of the present invention include those containing no adjuvant.

Furthermore, in the vaccine of the present invention, in addition to the above fusion protein as a vaccine antigen against a virus and/or a bacterium (preferably, SARS-CoV-2) that is a target for infection prevention and/or onset prevention, one or more vaccine antigens against a microorganism that causes another disease may be further blended.

A dosage form of the vaccine of the present invention is not particularly limited, and can be appropriately determined based on an administration method, storage conditions, and the like. Specific examples of the dosage form include liquid preparations and solid preparations, and more specifically, oral administration agents such as tablets, capsules, powders, granules, pills, solutions, and syrups; and parenteral administration agents such as injections and sprays.

A method for administering the vaccine of the present invention is not particularly limited, and examples thereof include injection administration such as intramuscular, intraperitoneal, intradermal, and subcutaneous administration; inhalation administration from the nasal cavity and the oral cavity; and oral administration. As described above, since the vaccine of the present invention does not require an adjuvant, it is particularly preferable that the vaccine is administered by inhalation administration from the nasal cavity, that is, by nasal administration, in view of the absence of an adjuvant to be used clinically in a vaccine for nasal administration.

A subject to which the vaccine of the present invention is applied may be a subject that should receive infection prevention and/or onset prevention against a virus and/or a bacterium, and a subject as an antigen having antibodies in the body against a virus and/or a bacterium different from the virus and/or bacterium against which infection prevention and/or onset prevention should be performed, and is preferably a subject that can develop SARS-CoV-2 symptoms by COVID-19 virus infection and has antibodies against an influenza virus. Examples of the influenza virus include a virus, which is the source of hemagglutinin and/or neuraminidase, components of the fusion protein. Examples of animals as such subjects to which the vaccine of the present invention is applied include mammals, and more specifically, humans; pet animals such as dogs and cats; and experimental animals such as rats, mice, and hamsters. In particular, in humans, since many of them have a history of influenza vaccination and/or a history of infection with influenza, the vaccine of the present invention has high versatility for humans.

A dose of the vaccine of the present invention is not particularly limited, and can be appropriately determined according to the type of an active ingredient, an administration method, and a subject receiving administration (conditions such as age, body weight, sex, and presence or absence of underlying disease).

The vaccine of the present invention can be used for the purpose of, for example, preventing the onset of an infection (particularly preferably COVID-19) by a virus and/or a bacterium that is a target for infection prevention, preventing and minimizing symptoms and pathological conditions associated with the infection, and treating and reducing the symptoms and the pathological conditions, and preferably, the vaccine can be used for the purpose of the prevention and minimization described above.

### EXAMPLES

Hereinafter, the present invention will be described in detail with reference to Examples, but the present invention is not limited thereto. The amino acid sequence of the RBD of SARS-CoV-2 used in the following Test Examples is an amino acid sequence from position 319 to 541 of SEQ ID NO: 1, the amino acid sequence of hemagglutinin is an amino acid sequence from position 18 to 523 of SEQ ID NO: 2, and the sequence of the linker peptide is one in which n = 1 and m = 1 in the above Linker Example 1.

In addition, in the following Test Examples, signs and abbreviations are used in the following meanings unless otherwise specified.

**[Table 1]**

| | |
|---|---|
| Mouse | 6-week-old male C57BL6 mouse |
| Cal7 | H1N1 influenza A virus (strain name: A/California/7/2009) |
| HA | A/California/7/2009-derived hemagglutinin |
| alum | Aluminum hydroxide |
| RBD | Recombinant protein of receptor-binding domain of SARS-CoV-2-derived S protein |
| RBD-HA | Fusion recombinant protein of the above RBD and the above HA |
| RBD+HA | Mixture of recombinant protein of the above RBD and the above HA |
| RBD+alum | Mixture of recombinant protein of the above RBD and aluminum oxide |
| RBD+c-di-GMP | Mixture of recombinant protein of the above RBD and c-di-GMP |
| RBD+CpG nucleic acid | Mixture of recombinant protein of the above RBD and CpG nucleic acid |
| RBD+AddaVax | Mixture of recombinant protein of the above RBD and AddaVax (InvivoGen) |
| RBD+poly(I:C) | Mixture of recombinant protein of the above RBD and poly(I:C) (doublestranded RNA analog) |

| | |
|---|---|
| alum, c-di-GMP, CpG nucleic acid, AddaVax, and poly(I:C) were used as adjuvants. | |

### Test Example 1

### (1) Experimental animals, reagents, and the like

- Mouse: 6-week-old male C57BL6 mouse
- Virus: H1N1 influenza A virus (strain: A/California/7/2009 (Cal7))
- Antigen (for comparison): Recombinant protein of receptor-binding domain (RBD) of SARS-CoV-2-derived S protein
- Antigen: Fusion recombinant protein of RBD and Cal7-derived hemagglutinin (HA) (RBD-HA)
- Adjuvant: Aluminum hydroxide

### (2) Experimental Methods

The upper respiratory tracts of mice were infected with Cal7, creating a situation in which the mice have antibodies against influenza viruses, as well as adults. After 30 days and 51 days from infection, the mice were subcutaneously immunized with RBD or RBD-HA. After 58 days from infection, blood was collected, and RBD-specific IgG in plasma was evaluated by ELISA.

### (3) Results

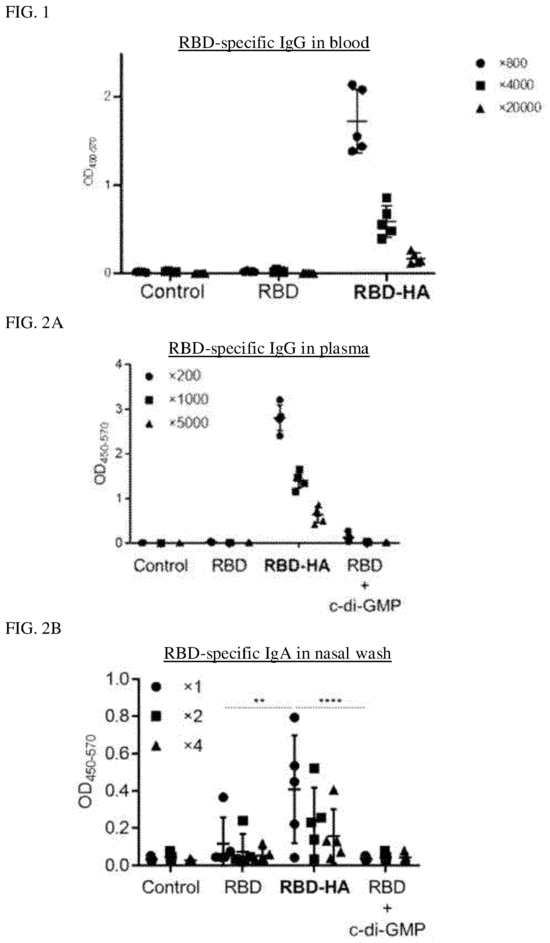

Measurement results of RBD-specific IgG in plasma are shown in Fig. 1. In the case of subcutaneous immunization with RBD after infection with Cal7 (RBD), production of antibody titers was not observed at all. On the other hand, in the case of immunization with RBD-HA after infection with Cal7 (RBD-HA), an RBD-specific antibody titer was observed. It was also confirmed that even when naive mice not infected with Cal7 were immunized with RBD-HA, no RBD-specific antibody titer was produced. In other words, it was suggested that HA-specific immune response induced by Cal7 infection contributes to strong immune induction of RBD-HA.

### Test Example 2

### (1) Experimental animals, reagents, and the like

- Mouse: 6-week-old male C57BL6 mouse
- Virus: H1N1 influenza A virus (strain: A/California/7/2009 (Cal7))
- Antigen (for comparison): Recombinant protein of receptor-binding domain (RBD) of SARS-CoV-2-derived S protein
- Antigen: Fusion recombinant protein of RBD and Cal7-derived hemagglutinin (HA) (RBD-HA)
- Adjuvant: c-di-GMP

### (2) Experimental Methods

The upper respiratory tracts of mice were infected with Cal7. After 30 days and 51 days from infection, the mice were nasally immunized with RBD or RBD-HA, or were nasally immunized with RBD together with c-di-GMP (RBD + c-di-GMP). After 58 days from infection, the nasal wash and blood were collected, and RBD-specific IgA in the nasal wash and RBD-specific IgG in plasma were evaluated by ELISA.

### (3) Results

Measurement results of RBD-specific IgA in the nasal wash and RBD-specific IgG in plasma are shown in Figs. 2A and 2B, respectively. In the figure, "**" represents p < 0.01, and "****" represents p < 0.0001. In the case where mice were nasally immunized with RBD after infection with Cal7 (RBD), production of antibody titers was not observed at all, and also in the case where c-di-GMP as an adjuvant was used in combination (RBD + c-di-GMP), no antibody titer was produced at all. On the other hand, in the case where mice were immunized with RBD-HA after infection with Cal7 (RBD-HA), RBD-specific IgA in the nasal wash and RBD-specific IgG in plasma were strongly induced even without using an adjuvant. From the above results, it was considered that RBD-HA can be an excellent vaccine antigen.

### Test Example 3

Mice were subcutaneously immunized with HA together with alum twice (Day 0 and Day 14) every 2 weeks to prepare HA-immunized mice. Mice were subcutaneously immunized with each of RBD and RBD-HA on Day 21 and Day 42. On Day 49, blood was collected, and RBD-specific IgG in plasma was evaluated by ELISA.

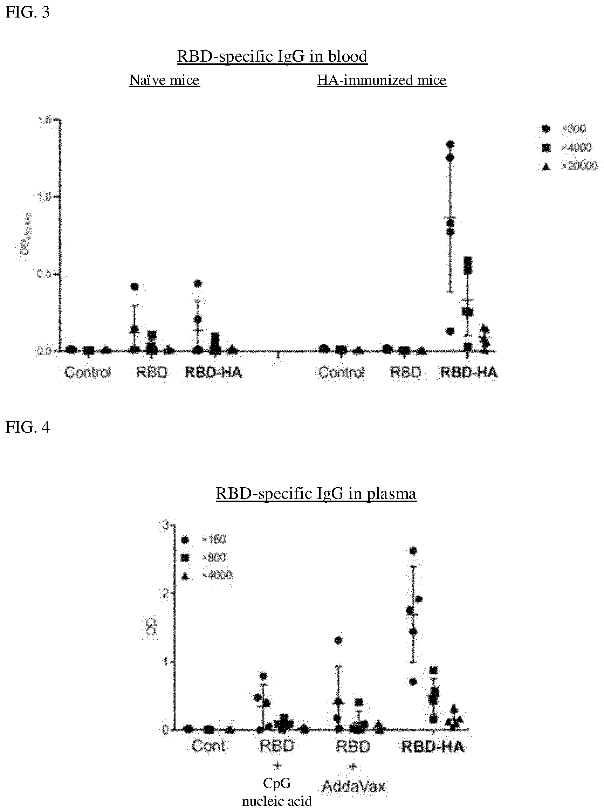

Results are shown in Fig. 3. Even when naive mice not immunized with HA were immunized with RBD-HA, no RBD-specific antibody titer was produced, suggesting that anti-HA antibodies contributes to immune induction of RBD-HA.

### Test Example 4

Mice were subcutaneously immunized with HA together with alum twice (Day 0 and Day 14) every 2 weeks. On Day 21, mice were subcutaneously immunized with each of RBD + CpG nucleic acid, RBD + AddaVax, and RBD-HA. On Day 49, blood was collected, and RBD-specific IgG in plasma was evaluated by ELISA.

Results are shown in Fig. 4. In the RBD-HA group without an adjuvant, an RBD-specific antibody with a higher intensity was induced than that in the RBD + CpG nucleic acid group and the RBD + AddaVax group with an adjuvant.

### Test Example 5

In the plasma sample of Test Example 1, neutralizing activity against SARS-CoV-2 was evaluated using pseudoviruses expressing SARS-CoV-2-derived S protein.

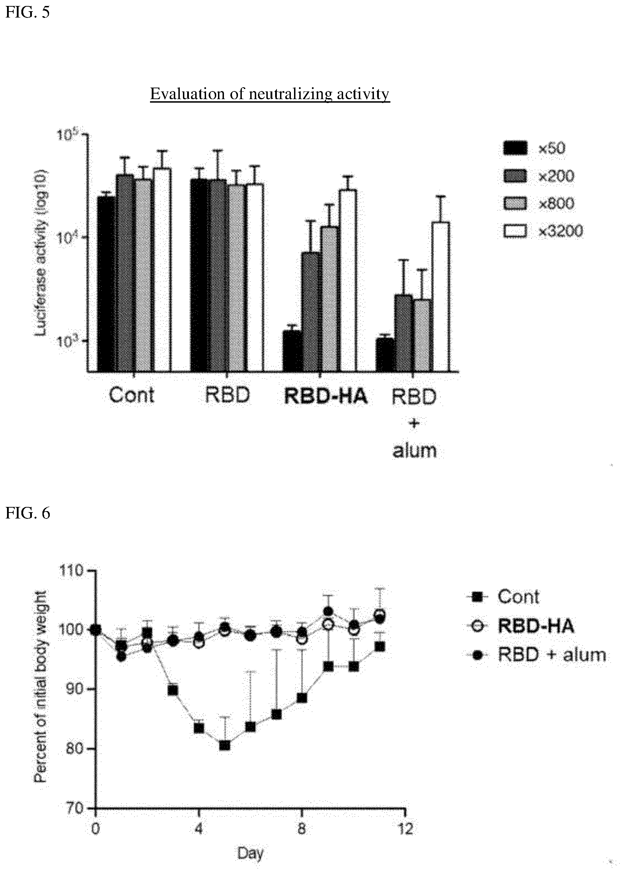

Results are shown in Fig. 5. It was found that the RBD-HA group even without an adjuvant can induce neutralizing antibodies equivalent to that of the RBD + alum group with an adjuvant.

### Test Example 6

The upper respiratory tracts of mice were infected with Cal7. After 30 days from infection, the mice were subcutaneously immunized with each of RBD-HA and RBD + alum. After 65 days from infection, the mice were infected with SARS-CoV-2 through the lower respiratory tracts from the nose (20 µL), and changes in body weight were evaluated.

Results are shown in Fig. 6. Body weight loss was observed in the non-immunized control group (Cont). No body weight loss was observed in the RBD + alum group and the RBD-HA group.

### Test Example 7

The upper respiratory tracts of mice were infected with Cal7. After 30 days from infection, the mice were subcutaneously immunized with each of RBD, RBD-HA, and RBD + HA. Fourteen days later, blood was collected, and RBD-specific IgG in plasma was evaluated by ELISA.

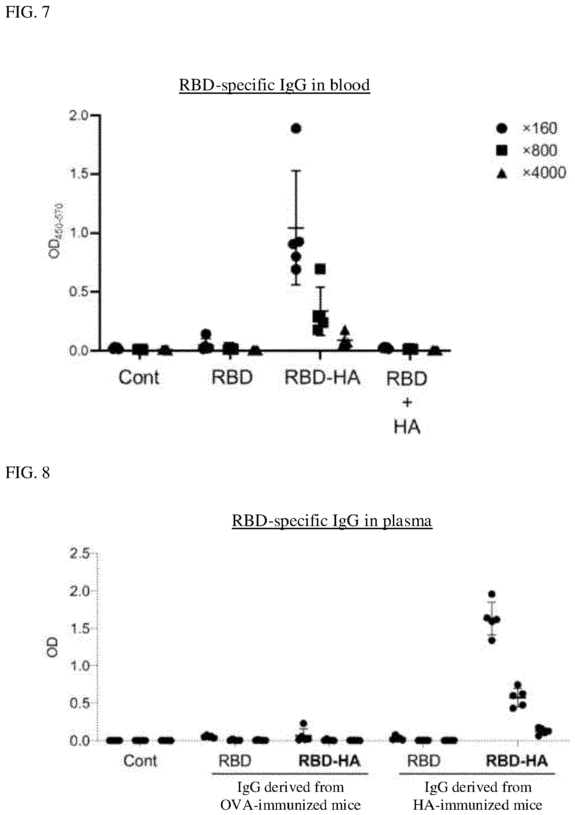

Results are shown in Fig. 7. An RBD-specific antibody titer was observed only in the RBD-HA group. This suggested that RBD could not be delivered to dendritic cells only by mixing RBD and HA, even if anti-HA antibodies were present.

### Test Example 8

Mice were immunized with ovalbumin (OVA) or HA together with aluminum hydroxide twice, and IgG was purified from mouse serum. The obtained purified IgG was mixed with RBD or RBD-HA, and the mixture was subcutaneously administered to naive mice. Fourteen days later, blood was collected, and RBD-specific IgG in plasma was evaluated by ELISA.

Results are shown in Fig. 8. Even when IgG purified from mice immunized with OVA was administered to mice together with RBD or RBD-HA, no increase in RBD-specific antibody titer was observed. Also when IgG purified from mice immunized with HA was administered together with RBD, no increase in RBD-specific antibody titer was observed. On the other hand, when IgG purified from mice immunized with HA was administered together with RBD-HA, a remarkable increase in RBD-specific antibody titer was observed. In consideration of the results of Test Example 7, it was shown that when RBD-HA is used for immune induction, immunity is induced due to the presence of anti-HA antibodies induced by HA immunization or influenza virus infection.

### Test Example 9

The upper respiratory tracts of mice were infected with Cal7. After 30 days and 51 days from infection, the mice were nasally immunized with each of RBD, RBD-HA, RBD + c-di-GMP, RBD + CpG nucleic acid, and RBD + poly(I:C) (6 µL). After 58 days from infection, blood was collected, and RBD-specific IgG in plasma was evaluated by ELISA.

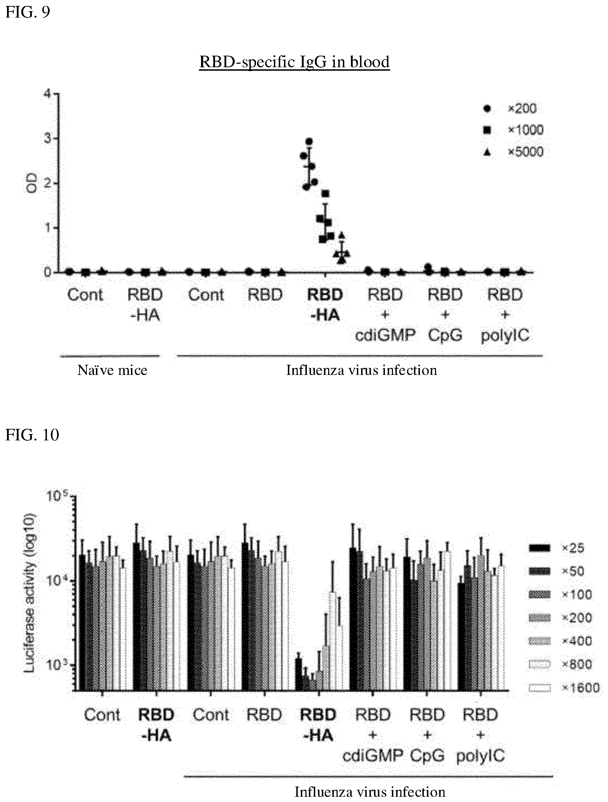

Results are shown in Fig. 9. Production of antibody titers was not observed in the RBD group. Also in the RBD + c-di-GMP group, the RBD + CpG nucleic acid group, and the RBD + poly(I:C) group in which an adjuvant was used in combination, no RBD-specific IgG in plasma was produced. On the other hand, in the RBD-HA group, RBD-specific IgG in plasma was strongly induced even without using an adjuvant. From this, RBD-HA can be an excellent antigen in a nasal vaccine.

### Test Example 10

In the plasma sample of Test Example 9, neutralizing activity was evaluated using pseudoviruses expressing SARS-CoV-2-derived S protein.

Results are shown in Fig. 10. Neutralizing activity was observed only in the RBD-HA group.

### Test Example 11

The upper respiratory tracts of mice were infected with Cal7. After 30 days and 51 days from infection, the mice were nasally immunized with each of RBD, RBD-HA, RBD + c-di-GMP, RBD + CpG nucleic acid, and RBD + poly(I:C) (6 µL). After 65 days from infection, the nasal wash was collected, and RBD-specific IgA in the nasal wash was evaluated by ELISA.

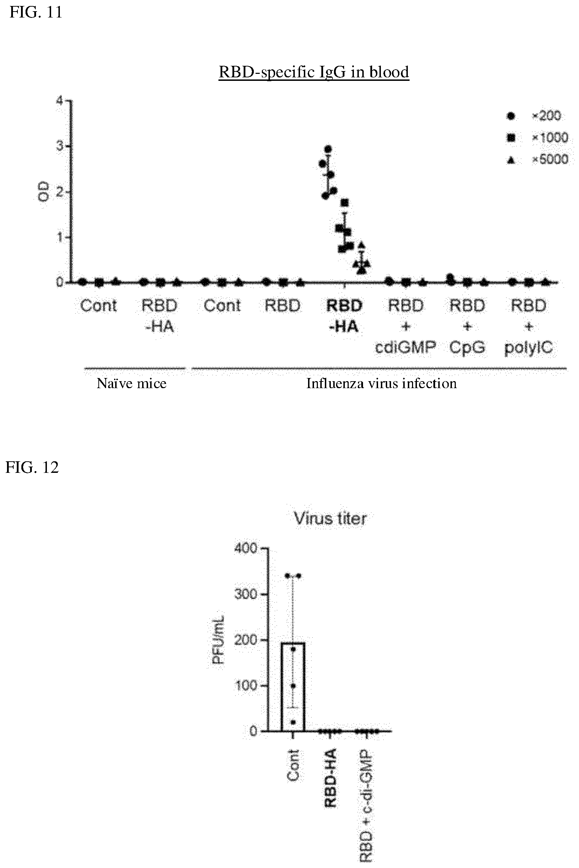

Results are shown in Fig. 11. As in the results of Test Examples 9 and 10, RBD-specific IgA was observed in the nasal wash only in the RBD-HA group.

### Test Example 12

The upper respiratory tracts of mice were infected with Cal7. After 30 days and 51 days from infection, the mice were nasally immunized with each of RBD-HA and RBD + c-di-GMP (6 µL). After 65 days from infection, the mice were infected with SARS-CoV-2 (5 µL) through the upper respiratory tract from the nose, and 3 days later, SARS-CoV-2 in the nasal wash was measured.

Results are shown in Fig. 12. In the control group that was only infected with Cal7, SARS-CoV-2 was observed in the nasal wash. On the other hand, SARS-CoV-2 was not observed in the RBD + c-di-GMP group or the RBD-HA group. From this, it was shown that RBD-HA can protect against SARS-CoV-2 in the upper respiratory tract without adding an adjuvant such as c-di-GMP.

### Test Example 13

The upper respiratory tracts of mice were infected with Cal7. After 30 days and 51 days from infection, the mice were nasally immunized with each of RBD, RBD-HA, and RBD+HA (6 µL). After 65 days from infection, the nasal wash was collected, and RBD-specific IgA in the nasal wash was evaluated by ELISA.

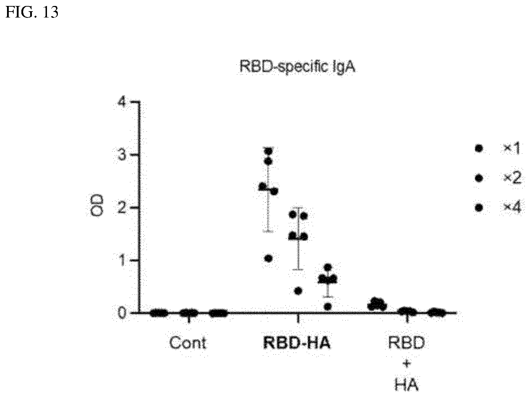

Results are shown in Fig. 13. RBD-specific IgA in the nasal wash observed in the RBD-HA group was not observed in the RBD + HA group. From this, it was considered that RBD could not be delivered to dendritic cells only by mixing RBD and HA, even if anti-HA antibodies were present. Thus, it was shown that RBD and HA need to be fused.

## Claims

1. A fusion protein comprising a receptor-binding domain of SARS-CoV-2 and hemagglutinin and/or neuraminidase.

2. The fusion protein according to claim 1, wherein the hemagglutinin is H1 type, H3 type, H5 type, and/or H7 type.

3. The fusion protein according to claim 1 or 2, which is used as a vaccine antigen against SARS-CoV-2.

4. A fusion protein that is used as a vaccine antigen and comprising an antigen of a virus and/or a bacterium and an antigen against which a vaccinated subject has antibodies in a body.

5. A vaccine comprising the fusion protein according to any one of claims 1 to 4.

6. The vaccine according to claim 5, which does not comprise an adjuvant.

7. The vaccine according to claim 5 or 6, which is administered to a subject having antibodies against an influenza virus.

8. The vaccine according to any one of claims 5 to 7, which is administered by injection.

9. The vaccine according to any one of claims 5 to 7, which is administered nasally.
